# EUROPEAN PATENT SPECIFICATION

(11) **EP 3 072 511 B1**
(45) Date of publication and mention of the grant of the patent: **13.12.2017**
(21) Application number: 14167142.0
(22) Date of filing: 06.05.2014
(51) Int. Cl.: A61K 31/122, A61K 31/357, A61K 31/662, A61P 35/00, A61P 35/02

(54) **The medical use of 3-aryl substituted 1-indanones**
Medizinische Anwendung 3-aryl substituierten 1-indanones
Utilisation médicale de 1-indanones, 3-aryl substituées

(30) Priority: 27.06.2013 PL 40448513
(43) Date of publication of application: 28.09.2016
(73) Proprietor: Centrum Badan Molekularnych I Makromolekularnych Polskiej Akademii Nauk, 90-363 Lodz (PL)
(72) Inventor: Balczewski, Piotr, 91-225 Lódz (PL); Szczesna, Dorota, 99-232 Zadzim (PL); Nawrot, Barbara, 95-100 Dabrówka (PL); Cieslak, Marcin, 93-640 Lódz (PL); Kazmierczak-Baranska, Julia, 94-123 Lódz (PL)
(74) Representative: Brodowska, Iwona

(56) References cited:
- FR-A1- 2 838 432
- LAWRENCE N J ET AL: "The synthesis of indanones related to combretastatin A-4 via microwave-assisted Nazarov cyclization of chalcones", TETRAHEDRON LETTERS, PERGAMON, GB, vol. 47, no. 10, 6 March 2006 (2006-03-06) , pages 1637-1640, XP025003671, ISSN: 0040-4039, DOI: 10.1016/J.TETLET.2005.12.110 [retrieved on 2006-03-06]
- SAXENA H O ET AL: "Gallic acid-based indanone derivatives as anticancer agents", BIOORGANIC & MEDICINAL CHEMISTRY LETTERS, PERGAMON, AMSTERDAM, NL, vol. 18, no. 14, 15 July 2008 (2008-07-15) , pages 3914-3918, XP022852863, ISSN: 0960-894X, DOI: 10.1016/J.BMCL.2008.06.039 [retrieved on 2008-06-14]

## Description

This invention relates to 3-aryl-2-phosphoryl substituted derivatives of 1-indanones as cytotoxic compounds for use in the treatment of chronic myelogenous leukemia K562 and cervical cancer HeLa.

The derivatives of 1-indanones are represented by the general formula **1a, 1b, 1c**, **2a** and **2b** 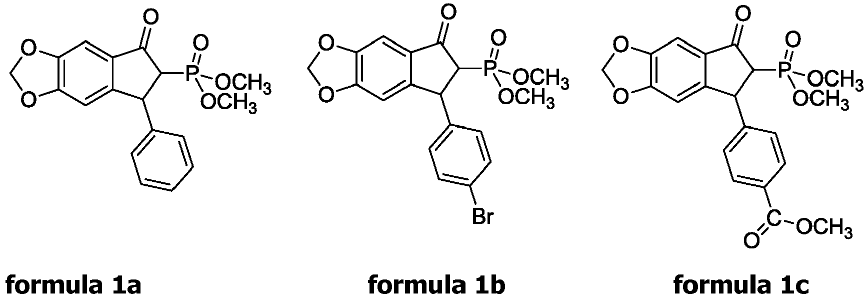 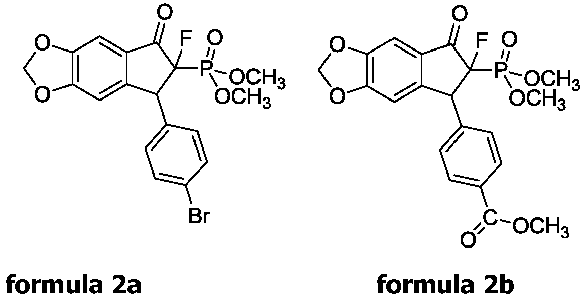 for use in the treatment of cervical cancer HeLa and chronic myelogenous leukemia K562.

### State of the Art

1-Indanones, and derivatives thereof are extensively used in agriculture, medicine and agrochemicals, while the 3-aryl-2-phosphoryl substituted derivatives of 1-indanones have not yet been tested for any of the biological properties.

1-Indanones exhibit a broad spectrum of biological activity. They are commonly used as medicines with: antiinflammatory [1], analgesic [2], and antimicrobial [3], antiviral [4], or antimalarial [5] and an antitumor [6] activity.

2-Benzylidene-3-aryl substituted 1-indanones, exhibiting a very high toxicity in relation to tumor cells of breast cancer, colon cancer, leukemia and lung cancer (MCF-7, HCT, THP-1, A549; IC₅₀=10-880 nM) and having the ability to inhibit tubulin polymerization (IC₅₀=0.62-2.04 µM, have been examined by A. P. Prakasham and A. K. Saxena [7].

High cytotoxicity towards the K562 cells (IC₅₀ of 0.10 to 2.0 µM) has been shown by 3-aryl substituted 1-indanones, lacking the phosphoryl group, obtained by cyclization of the appropriate chalcones in the presence of trifluoroacetic acid or involving microwave [8].

1-Indanones, unsubstituted in position 2 and 3, were also tested for relaxant effect on the smooth muscles of the blood vessels and the resulting values of EC₅₀ of 1.3×10⁻⁶ to 1.1×10⁻⁵M were three orders of magnitude higher than commonly used medicament Nifedipine [9].

3-Aryl-1-indanones were found as anticancer agents, in particular as inhibiting cell growth of K562 cell line (Lawrence, NJ et al, Tetrahedron Lett. 2006, 47, 1637-1640). 3-Aryl-1-indanones also were described as compounds inhibiting tumor cells proliferation and treating cancer, in particular HeLa cell proliferation (FR 2 838 432 A1 (Aventis Pharma SA), 17 October 2003).

Thiosemicarbazone derivatives of 1-indanones, unsubstituted in position 2 and 3, were tested for activity against bovine diarrhea virus, which was used as a counterpart of hepatitis virus C. A very good selectivity index (SI=80.29) was obtained, much higher than the selectivity index for the commonly used ribavirin (SI=11.64) [6].

Compounds of general **formula 1a, 1b, 1c, 2a** and **2b** and their synthesis have not been known so far (linking to the application P-404112 5.29.2013) and antitumor effect of these compounds, in particular activity towards cells of cervical cancer HeLa or tumor cells of chronic myelogenous leukemia K562 has not yet been reported.

Compound represented by the formula **1a, 1b, 1c,** 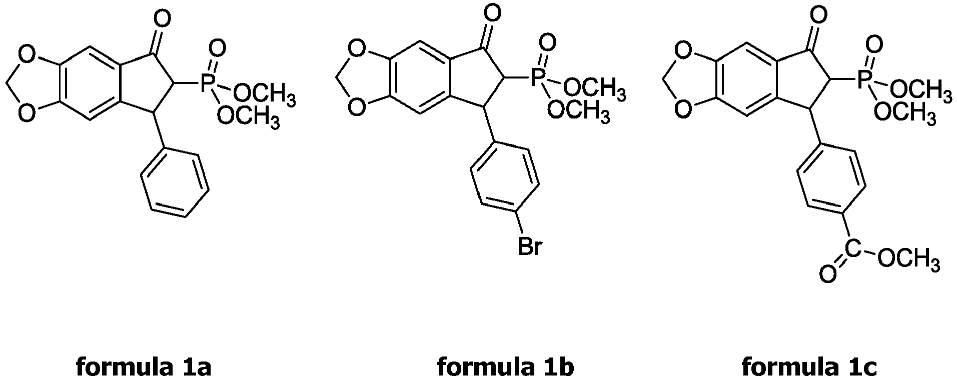 2-phosphoryl-3-phenyl substituted 1-indanone of the molecular formula C₁₈H₁₇O₆P **(1a),** 2-phosphoryl-3-(*para*-bromophenyl) substituted 1-indanone of the molecular formula C₁₈H₁₆BrO₆P **(1b),** 2-phosphoryl-3-(*para*-methoxycarbonyl)phenyl substituted 1-indanone of the molecular formula C₂₀H₁₉O₈P **(1c),** for use in the treatment of cervical cancer HeLa and chronic myelogenous leukemia K562.

Compound represented by the formula **2a** and **2b** 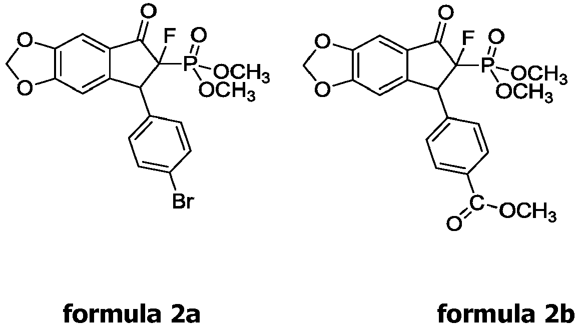 2-fluoro-2-phosphoryl-3-(*para*-bromophenyl) substituted 1-indanone of the molecular formula C₁₈H₁₅BrFO₆P **(2a)**, 2-fluoro-2-phosphoryl-3-(*para*-methoxycarbonyl)-phenyl substituted 1-indanone of the molecular formula C₂₀H₁₈FO₈P **(2b)**, for use in the treatment of cervical cancer HeLa and chronic myelogenous leukemia K562.

Compounds of the formula **1a, 1b** and **1c** have not been studied so far in terms of antitumor activity. Particularly, their activity against cells of cervical cancer HeLa and chronic myelogenous leukemia K562 hasn't been tested.

A compound of the formula **1a** shows significant cytotoxicity against cells of cervical cancer HeLa (IC₅₀=90µM after 48 h) and cytotoxicity against cells of chronic myelogenous leukemia K562 (IC₅₀=100µM after 48 h), and can therefore be used for the preparation of anticancer medicaments.

A compound of the formula **1b** shows significant cytotoxicity against cells of cervical cancer line HeLa (IC₅₀>100 µM after 48 h) and cytotoxicity against cells of chronic myelogenous leukemia K562 (IC₅₀=70µM after 48 h), and can therefore be used for the preparation of anticancer medicaments.

A compound of the formula **1c** shows significant cytotoxicity against cells of cervical cancer HeLa (IC₅₀>100 µM after 48 h) and cytotoxicity against cells of chronic myelogenous leukemia K562 (IC₅₀=100µM after 48 h), and can therefore be used for the preparation of anticancer medicaments.

According to the invention, the compounds of the formula **2a** and **2b,** substituted in the *para* position by a bromine atom or a methoxycarbonyl group, 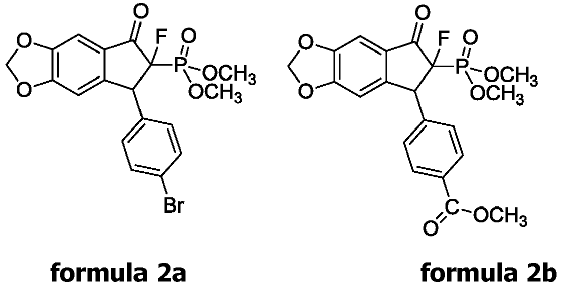 i.e. 2-fluoro-2-phosphoryl-3-(*para*-bromophenyl) substituted 1-indanone of the molecular formula C₁₈H₁₅BrFO₆P **(2a)**, 2-fluoro-2-phosphoryl-3-(*para-*methoxycarbonyl)-phenyl substituted 1-indanone of the molecular formula C₂₀H₁₈FO₈P **(2b)** having an antitumor activity against cells of cervical cancer HeLa and chronic myelogenous leukemia K562.

Compounds of the formula **2a** and **2b** have not been studied so far in terms of antitumor activity. In particular, their activity against cells of cervical cancer HeLa and chronic myelogenous leukemia K562 has not been tested.

A compound of the formula **2a** shows significant cytotoxicity against cells of cervical cancer HeLa (IC₅₀=70µM after 48 h) and cytotoxicity against cells of chronic myelogenous leukemia K562 (IC₅₀=60µM after 48 h), and can therefore be used for the preparation of anticancer medicaments.

A compound of the formula **2b** shows significant cytotoxicity against cells of cervical cancer HeLa (IC₅₀>100 µM after 48 h) and cytotoxicity against cells of chronic myelogenous leukemia K562 (IC₅₀=80µM after 48 h), and can therefore be used for the preparation of anticancer medicaments.

### EXAMPLE I

Compounds **1a-1c, 2a, 2b**, were tested for the cytotoxic properties against the two cell lines using the MTT test (yellow tetrazolium salt processed by dehydrogenases in the mitochondria of living cells into the purple formazan)
- cell line HeLa (cervix carcinoma, human)
- cell line K562 (chronic myelogenous leukemia, human).
1. For this purpose, HeLa cells and K562 cells in an amount of seven thousand cells/well were seeded in 96-well plates. Cells were incubated overnight in an incubator (37°C, 5% CO₂)
2. Solutions of test compounds in DMSO were prepared. Appropriate volumes of solutions were added into the cell culture medium, wherein the concentration of DMSO in the culture medium was a maximum of 1%. The final concentration of the test compounds in cell cultures was 1 mM, 1×10⁻¹ mM, 1×10⁻² mM, 1×10⁻³ mM, 1×10⁻⁴ mM, 1×10⁻⁵ mM. As a control, staurosporine (at 1µM final concentration in the cell culture, 1% DMSO) was used.
3. In order to perform the cytotoxicity test (MTT test) in the HeLa cells (or K562) after 48h incubation with the test compounds, water solution of MTT (5mg/ml) was added in an amount of 25 µl/well (MTT, a yellow tetrazolium salt is reduced to purple formazan by mitochondrial dehydrogenases of living cells, the quantity of which can be determined spectrophotometrically). Cells were incubated with MTT for 2h in an incubator (37°C, 5% CO₂). After 2h incubation, a lysis buffer containing SDS and DMF was added to the cell culture (95 µl/well) and incubated overnight (37°C, 5% CO₂).
4. MTT test plates were analyzed by reading the absorbance at a wavelength of 570 nm and 650 nm on a Fluostar Omega reader. The absorbance measured at 570 nm was proportional to the number of viable cells. IC₅₀ values were read from the graphs by interpolation for 50% cell survival.
5. The obtained results, which indicate the toxicity of compounds **1a-1c, 2a**, **2b**, against adherent cells and suspension cells are shown in Table 1

**Table 1**

| Compound | HeLa IC₅₀ after 48h | K562 IC₅₀ after 48h |
|---|---|---|
| **1a** | 90 µM | 100 µM |
| **1b** | > 100 µM | 70 µM |
| **1c** | > 100 µm | 100 µM |
| **2a** | 70 µM | 60 µM |
| **2b** | > 100 µm | 80 µM |
| | | |

After 48 hours of use of the compound, a strong toxicity against cervical cancer cells (HeLa) is observed. A similar effect was observed against chronic myelogenous leukemia cells.

### Literature:

[1] V. S. Saravanan, P. S. Selvan, n. Gopal, J. K. Gupta, Asian J. Chem. 2006, 18, 2597-2604
[2] P. D. Hammen, G. M. US Patent, 1979, 4, 164, 514
[3] R. Albrecht, H.J. Kessler, E. Schroder, US Patent, 1972, 3, 671, 520
[4] L. M. Finkieisztein, E. Y. Moltrasio, R. H. Campos, E. F. Castro, L. E. Fabian, Eur. J. Med. Chem, 2008, 43, 1767-1773
[5] J. E. Charris, G. M. Lobo, J. Camacho, R. Ferrer, A. Barazarte, J. N. Dominguez, Lett. Drug. Des. Discov. 2007, 4, 49-54
[6] H. O. Saxena, U. Faridi, S. Srivastava, J. K. Kumar, Bioorg. Med. Chem. Lett., 2008, 18, 3914-3918
[7] A.P. Prakasham, A. K. Saxena, S. Lugman, D. Chanda, Bioorg. Med. Chem., 2012, 20, 3049-3057
[8] N. J. Lawrence, E. S. M. Armitage, B. Greedy, D. Cook, S. Ducki, A. T. McGown, Tetrahedron Letters, 2006, 47, 1637-1640
[9] H. Sheridan, N. Frankish, R. Farrell, Eur. J. Med. Chem. 1999, 34, 953-966

## Claims

1. Compound of the formula **1a, 1b** or **1c** 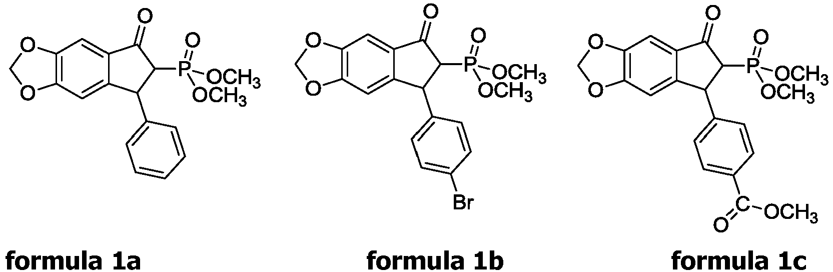 i.e. 2-phosphoryl-3-phenyl substituted 1-indanone of the molecular formula C₁₈H₁₇O₆P **(1a),** 2-phosphoryl-3-(*para*-bromophenyl) substituted 1-indanone of the molecular formula C₁₈H₁₆BrO₆P **(1b),** 2-phosphoryl-3-(*para-*methoxycarbonyl)phenyl substituted 1-indanone of the molecular formula C₂₀H₁₉O₈P **(1c)** for use in the treatment of cervical cancer HeLa cells and chronic myelogenous leukemia K562 cells.

2. Compound of the formula **2a** or **2b** 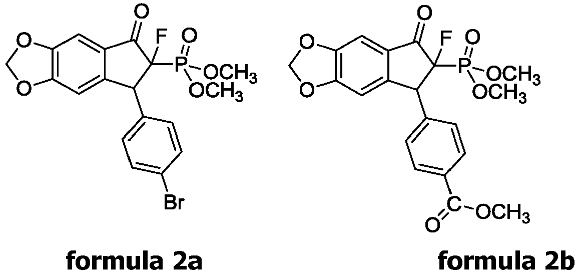 i.e. 2-fluoro-2-phosphoryl-3-(*para*-bromophenyl) substituted 1-indanone of the molecular formula C₁₈H₁₅BrFO₆P **(2a)**, 2-fluoro-2-phosphoryl-3-(*para-*carbonylmethoxy)-phenyl substituted 1-indanone of the molecular formula C₂₀H₁₈FO₈P **(2b)** for use in the treatment of cervical cancer HeLa cells and chronic myelogenous leukemia K562 cells.

## Patentansprüche

1. Zusammensetzung der Formel **1a, 1b** oder **1c** 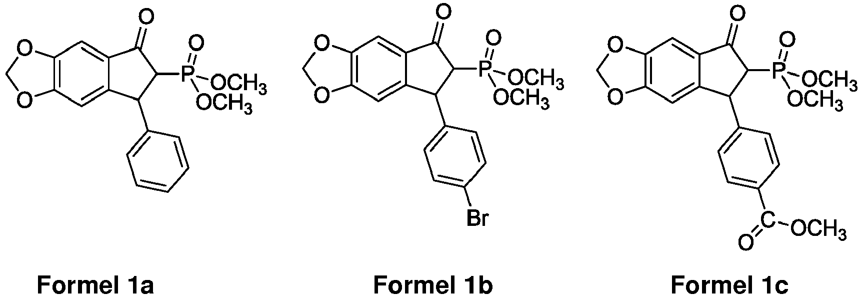 2-phosphoryl-3-phenyl substituierte 1-Indanon der Molekularformel C18H17O6P (1a), 2-Phosphoryl-3-(para-Bromphenyl) substituierte 1-Indanon der Molekularformel C₁₈H₁₆BrO₆P (1b), 2-Phosphoryl-3- (para-methoxycarbonyl) phenyl-substituierte 1-Indanon der molekularen Formel C20H1908P (1c) zur Verwendung bei der Behandlung von Gebärmutterhalskrebs-HeLa und Zellen der chronischen myeloischen Leukämie K562.

2. Zusammensetzung der Formel **2a** oder **2b** 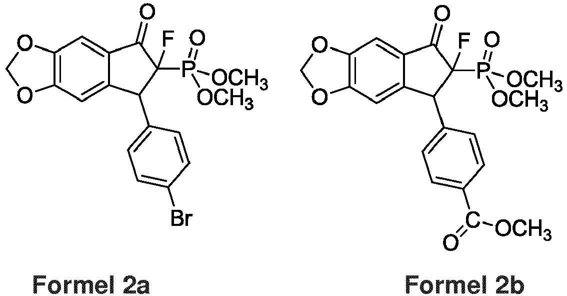 2-fluor-2-phosphoryl-3-(para-Bromphenyl) substituierte 1-Indanon der Molekularformel C18H15BrFO6P (2a), 2-Fluor-2-phosphoryl-3- (para-methoxycarbonyl) phenyl-substituierte 1-Indanon von der molekularen Formel C20H18FO8P (2b) zur Verwendung bei der Behandlung von Gebärmutterhalskrebs-HeLa-Zellen und chronischen myeloischen Leukämie-K562-Zellen.

## Revendications

1. Composé de formule **1a**, **1b** ou **1c** 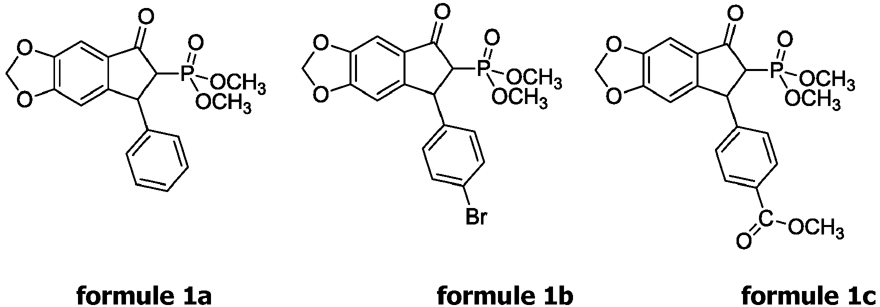 2-phosphoryl-3-phényl substitué 1-indanone de formule moléculaire C₁₈H₁₇O₆P **(1a),** 2-phosphoryl-3- (para-bromophényl) substitué 1-indanone de formule moléculaire C₁₈H₁₆BrO₆P **(1b),** 2-phosphoryl-3- (1-indanone substituée par para-méthoxycarbonyl) phényle de formule moléculaire C₂₀H₁₉O₈P **(1c)** destinée à être utilisée dans le traitement du cancer du col de l'utérus HeLa et des cellules de la leucémie myéloïde chronique K562.

2. Composé de formule **2a** ou **2b** 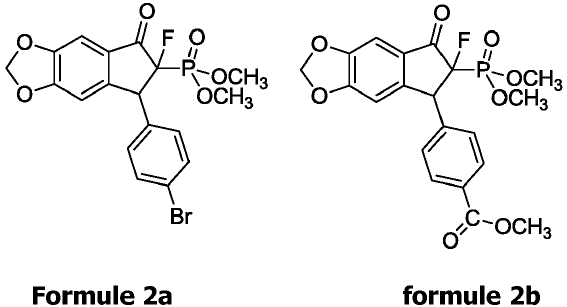 2-fluoro-2-phosphoryl-3- (para-bromophényl) substitué 1-indanone de formule moléculaire C₁₈H₁₅BrFO₆P **(2a)**, 2-fluoro-2-phosphoryl-3- (para-carbonylméthoxy) - phényle substitué 1-indanone de la formule moléculaire C₂₀H₁₈FO₈P **(2b)** destinée à être utilisée dans le traitement des cellules HeLa du cancer du col de l'utérus et des cellules de la leucémie myéloïde chronique K562.
